(19) Europäisches Patentamt
European Patent Office
Office européen des brevets

(11) EP 4 726 025 A1

(12) EUROPEAN PATENT APPLICATION
published in accordance with Art. 153(4) EPC

(43) Date of publication:
15.04.2026 Bulletin 2026/16

(21) Application number: 25784935.6

(22) Date of filing: 13.05.2025

(51) International Patent Classification (IPC):
C12N 1/20 (2026.01)          A23C 9/123 (2006.01)
A61K 35/745 (2015.01)        A61P 1/00 (2006.01)

(86) International application number:
PCT/CN2025/094593

(87) International publication number:
WO 2026/040497 (26.02.2026 Gazette 2026/09)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 21.08.2024 CN 202411148077

(71) Applicant: Wecare Probiotics Co., Ltd.
Suzhou, Jiangsu 215200 (CN)

(72) Inventors:
• FANG, Shuguang
Suzhou, Jiangsu 215200 (CN)

• WU, Yanfeng
Suzhou, Jiangsu 215200 (CN)
• KONG, Sufen
Suzhou, Jiangsu 215200 (CN)
• CHEN, Xueqin
Suzhou, Jiangsu 215200 (CN)
• GUO, Xiaojuan
Suzhou, Jiangsu 215200 (CN)
• ZHU, Jianguo
Suzhou, Jiangsu 215200 (CN)

(74) Representative: Buzzi, Notaro & Antonielli d'Oulx
S.p.A.
Corso Vittorio Emanuele II, 6
10123 Torino (IT)

(54) COMPOSITE BIFIDOBACTERIUM PREPARATION AND USE THEREOF IN YOGURT FERMENTATION

(57) Provided are a composite *Bifidobacterium* inoculant and a use thereof in yogurt fermentation. A strain in the composite *Bifidobacterium* inoculant is composed of a *Bifidobacterium animalis* subsp. *lactis* BLa36 strain, a *Bifidobacterium longum* BL21 strain, a *Bifidobacterium breve* BBr60 strain, a *Bifidobacterium adolescentis* BAC30 strain and a *Bifidobacterium infantis* BI45 strain. The five particular different types of *Bifidobacterium* strains are compounded. With potential interactions, the five strains can cooperate with each other and achieve a synergistic effect in improving the tolerance of the strains to gastric juice and an overall survival rate of fermented strains. Combining the five strains with basic fermented strains for yogurt fermentation can significantly improve the acid tolerance of *Lactobacillus* in yogurt, significantly increase the number of viable bacteria entering the human intestinal tract and playing a role, significantly promote intestinal peristalsis, promote gastric motility, improve an imbalance in intestinal flora and promote intestinal health.

FIG. 1

## Description

TECHNICAL FIELD

The present application belongs to the technical field of probiotic agents and relates to a composite *Bifidobacterium* inoculant and a use thereof in yogurt fermentation.

BACKGROUND

A wide variety of microorganisms residing in the intestinal tract are important participants in metabolism of the host and play an important role in the aspects such as food digestion and nutrient absorption, immune regulation and disease defense of the host. The intake of a sufficient amount of probiotics can have a beneficial effect on the health of the host. Probiotics produce beneficial metabolites and inhibit the overgrowth of harmful bacteria, promoting the balance of the intestinal environment. Therefore, additional supplementation of probiotics is considered as an important manner to restore intestinal balance. The disruption to the intestinal microecological balance may cause symptoms such as irritable bowel syndrome, constipation and diarrhea.

Yogurt is a good carrier for probiotics, and ordinary yogurt generally contains only the basic fermentation bacterial strains-*Streptococcus salivarius* subsp. *thermophilus* and *Lactobacillus delbrueckii* subsp. *bulgaricus.* Although these bacterial strains play a certain role in the fermentation process, their effects are relatively limited. The main reason is that *Streptococcus salivarius* subsp. *thermophilus* and *Lactobacillus delbrueckii* subsp. *Bulgaricus* are difficult to tolerate a gastric acid environment with a low pH, resulting in the extremely limited number of viable bacteria of *Streptococcus salivarius* subsp. *thermophilus* and *Lactobacillus delbrueckii* subsp. *Bulgaricus* entering the human intestinal tract and playing a role. However, the addition of probiotics to yogurt is an important manner to increase the number of beneficial viable bacteria of yogurt in the intestinal tract.

To improve the probiotic supplementation effect of yogurt, attempts are made to develop more types of acid-tolerable probiotics such as *Bifidobacterium, Lactobacillus acidophilus* and *Lactobacillus rhamnosus.* With stronger acid tolerance, these probiotics can better resist the erosion of gastric acid and bile, increasing their survival rate of reaching the intestinal tract. Moreover, the problems of flavor imbalance and non-ideal texture that probiotics may bring to yogurt are also considered. The addition of high doses of probiotics to yogurt has been facing major challenges.

SUMMARY

The present application provides a composite *Bifidobacterium* inoculant and a use thereof in yogurt fermentation.

In a first aspect, the present application provides a composite *Bifidobacterium* inoculant. A strain in the composite *Bifidobacterium* inoculant is composed of a *Bifidobacterium animalis* subsp. *lactis* BLa36 strain with a deposit number CGMCC No. 24029, *a Bifidobacterium longum* BL21 strain with a deposit number CGMCC No. 10452, a *Bifidobacterium breve* BBr60 strain with a deposit number CGMCC No. 12915, a *Bifidobacterium adolescentis* BAC30 strain with a deposit number CGMCC No. 19884 and a *Bifidobacterium infantis* BI45 strain with a deposit number CGMCC No. 15134.

The present application develops a brand-new probiotic compounding manner which is to compound the five particular different types of *Bifidobacterium* strains. It is found that with potential interactions, the five strains can cooperate with each other and achieve a synergistic effect in improving the tolerance of the strains to gastric juice and an overall survival rate of fermented strains. Combining the five strains with basic fermented strains for yogurt fermentation can significantly improve the acid tolerance of *Lactobacillus* in yogurt and significantly increase the number of viable bacteria entering the human intestinal tract and playing a role. Moreover, the prepared yogurt has a smooth texture, a rich flavor and an intense fragrance and can significantly promote intestinal peristalsis, promote gastric motility, improve an imbalance in intestinal flora and promote intestinal health. In the case of consistent amounts of used bacteria, the composite *Bifidobacterium* inoculant involved in the present application is more excellent in exhibiting the above efficacy compared with other *Bifidobacterium* compounding manners.

Preferably, a ratio of the viable bacteria count of the BLa36 strain to the viable bacteria count of the BL21 strain to the viable bacteria count of the BBr60 strain to the viable bacteria count of the BAC30 strain to the viable bacteria count of the BI45 strain is (1-10):(1-10):(1-10):(1-5):(1-5).

1, 2, 3, 4, 5, 6, 7, 8, 9 or 10 may be selected as a specific point value within (1-10), and 1, 1.5, 2, 2.5, 3, 3.5, 4, 4.5 or 5 may be selected as a specific point value within (1-5). Other specific point values within the numerical ranges may be selected, and details are not described here.

Based on the potential mutual cooperation relationships among the five *Bifidobacteria,* the five *Bifidobacteria* have more prominent effects in improving the tolerance to the gastric juice and the overall survival rate of the strains, improving the texture, flavor and fragrance of yogurt, promoting the intestinal peristalsis and promoting the gastric motility when meeting the above particular ratio relationship of the viable bacteria count.

Preferably, a total viable bacteria count in the composite *Bifidobacterium* inoculant is not less than $1 \times 10^{11}$ CFU/mL or $1 \times 10^{11}$ CFU/g, for example, $1 \times 10^{11}$ CFU/mL (CFU/g), $5 \times 10^{11}$ CFU/mL (CFU/g), $1 \times 10^{12}$ CFU/mL (CFU/g) or $5 \times 10^{12}$ CFU/mL (CFU/g). Other specific point values within the numerical ranges may be selected, and details are not described here.

Preferably, a dosage form of the composite *Bifidobacterium* inoculant includes a solution, a freeze-dried powder, a capsule, a tablet or a granule.

When the composite *Bifidobacterium* inoculant is a freeze-dried powder, the composite *Bifidobacterium* inoculant is prepared through a preparation method including the following steps:
inoculating the relevant strains in media, respectively, for activation and fermentation culture to obtain fermentation broths; subjecting the fermentation broths to centrifugation respectively, mixing with a lyoprotectant, and performing lyophilization to obtain bacteria powders of each strain; and mixing the bacteria powders of each strain according to a ratio of the viable bacteria count to obtain the composite *Bifidobacterium* inoculant.

Preferably, the lyoprotectant includes any one or a combination of at least two of skimmed milk, gelatin, dextrin, arabic gum, dextran, sodium alginate, polyvinylpyrrolidone, sucrose, lactose, trehalose, sorbitol or xylitol.

In a second aspect, the present application provides a composite probiotic agent. The composite probiotic agent includes the composite *Bifidobacterium* inoculant described in the first aspect and a basic inoculant, where a strain in the basic inoculant is composed of a *Streptococcus thermophilus* ST81 strain with a deposit number CGMCC No. 15752 and a *Lactobacillus delbrueckii* subsp. *Bulgaricus* LB42 strain with a deposit number CGMCC No. 15751.

The present application also finds that combining the composite *Bifidobacterium* inoculant involved in the first aspect with the *Streptococcus thermophilus* ST81 strain and the *Lactobacillus delbrueckii* subsp. *Bulgaricus* LB42 strain for yogurt fermentation can significantly increase the total viable bacteria count of *Lactobacilli* in yogurt. The prepared yogurt has a smooth texture, a rich flavor and an intense fragrance and can significantly promote intestinal peristalsis, promote gastric motility, improve an imbalance in intestinal flora and promote intestinal health.

Preferably, a ratio of the viable bacteria count of the ST81 strain to the viable bacteria count of the LB42 strain is 30:(0.001-10), for example, 30:0.001, 30:0.01, 30:0.1, 30:0.5, 30:1, 30:2, 30:3, 30:4, 30:5, 30:6, 30:8 or 30:10. Other specific point values within the numerical range may be selected, and details are not described here.

The total viable bacteria count in the basic inoculant is not less than $1 \times 10^{11}$ CFU/mL or $1 \times 10^{11}$ CFU/g, for example, $1 \times 10^{11}$ CFU/mL (CFU/g), $5 \times 10^{11}$ CFU/mL (CFU/g) or $1 \times 10^{12}$ CFU/mL (CFU/g). Other specific point values within the numerical ranges may be selected, and details are not described here.

A mass ratio of the composite *Bifidobacterium* inoculant to the basic inoculant is (10-300):(10-40). 10, 20, 30, 50, 70, 80, 100, 150, 200, 250 or 300 may be selected as a specific point value within (10-300), and 10, 15, 20, 25, 30, 35 or 40 may be selected as a specific point value within (10-40). Other specific point values within the numerical ranges may be selected, and details are not described here.

A dosage form of the composite probiotic agent includes a solution, a freeze-dried powder, a capsule, a tablet or a granule.

In a third aspect, the present application provides a use of the composite *Bifidobacterium* inoculant described in the first aspect or the composite probiotic agent described in the second aspect in yogurt fermentation.

Preferably, a method of the yogurt fermentation includes:

(1) mixing raw cow milk with composite prebiotics, sterilizing and cooling the mixture, and using the mixture as a fermentation base; and

(2) inoculating the composite probiotic agent in the fermentation base for fermentation.

Preferably, the composite prebiotics include any one or a combination of at least two of fructooligosaccharides, galactooligosaccharides, xylooligosaccharides, isomaltooligosaccharides, soybean oligosaccharides, inulins, polydextrose, α-whey protein or lactoferrin.

Preferably, the composite prebiotics are 0.01% to 1% of the fermentation base, for example, 0.01%, 0.05%, 0.1%, 0.2%, 0.3%, 0.4%, 0.5%, 0.6%, 0.7%, 0.8%, 0.9% or 1%.

Preferably, an inoculation amount of the composite probiotic agent is 20-300 g/T, for example, 20 g/T, 50 g/T, 70 g/T, 80 g/T, 100 g/T, 150 g/T, 200 g/T, 250 g/T or 300 g/T.

Preferably, the fermentation is performed at a temperature of 40-43°C (for example, 40°C, 41°C, 42°C or 43°C) for 5-10 h (for example, 5 h, 6 h, 7 h, 8 h, 9 h or 10 h), and when an acidity reaches 70-80°T (for example, 70°T, 72°T, 75°T, 78°T or 80°T) or pH = 4.3-4.5 (for example, pH = 4.3, pH = 4.4 or pH = 4.5), the fermentation reaches an end point.

Other specific point values within the numerical ranges may be selected, and details are not described here.

In a fourth aspect, the present application provides a use of the composite *Bifidobacterium* inoculant described in the first aspect or the composite probiotic agent described in the second aspect in preparing a product for promoting intestinal peristalsis and promoting intestinal health.

The product includes a food, a health care product or a drug.

Compared with the prior art, the present application has the beneficial effects below.

The present application develops a brand-new probiotic compounding manner which is to compound the five particular different types of *Bifidobacterium* strains. It is found that with potential interactions, the five strains can cooperate with each other and achieve a synergistic effect in improving the tolerance of the strains to gastric juice and an overall survival rate of fermented strains. Combining the five strains with basic fermented strains for yogurt fermentation can significantly improve the acid tolerance of *Lactobacillus* in yogurt and significantly increase the number of viable bacteria entering the human intestinal tract and playing a role. Moreover, the prepared yogurt has a smooth texture, a rich flavor and an intense fragrance and can significantly promote intestinal peristalsis, promote gastric motility, improve an imbalance in intestinal flora and promote intestinal health. In the case of consistent amounts of used bacteria, the composite *Bifidobacterium* inoculant involved in the present application is more excellent in exhibiting the above efficacy compared with other *Bifidobacterium* compounding manners.

A classification of the BLa36 strain involved in the present application is named *Bifidobacterium animalis* subsp. *lactis* and deposited in the China General Microbiological Culture Collection Center on December 2, 2021, with a deposit number CGMCC No. 24029, where the China General Microbiological Culture Collection Center is located at No. 3, NO. 1 West Beichen Road, Chaoyang District, Beijing.

A classification of the BL21 strain involved in the present application is named *Bifidobacterium longum* and deposited in the China General Microbiological Culture Collection Center on January 27, 2015, with a deposit number CGMCC No. 10452, where the China General Microbiological Culture Collection Center is located at No. 3, NO. 1 West Beichen Road, Chaoyang District, Beijing.

A classification of the BBr60 strain involved in the present application is named *Bifidobacterium breve* and deposited in the China General Microbiological Culture Collection Center on August 29, 2016, with a deposit number CGMCC No. 12915, where the China General Microbiological Culture Collection Center is located at No. 3, NO. 1 West Beichen Road, Chaoyang District, Beijing.

A classification of the BAC30 strain involved in the present application is named *Bifidobacterium adolescentis* and deposited in the China General Microbiological Culture Collection Center on May 28, 2020, with a deposit number CGMCC No. 19884, where the China General Microbiological Culture Collection Center is located at No. 3, NO. 1 West Beichen Road, Chaoyang District, Beijing.

A classification of the BI45 strain involved in the present application is named *Bifidobacterium infantis* and deposited in the China General Microbiological Culture Collection Center on December 27, 2017, with a deposit number CGMCC No. 15134, where the China General Microbiological Culture Collection Center is located at No. 3, NO. 1 West Beichen Road, Chaoyang District, Beijing.

A classification of the ST81 strain involved in the present application is named *Streptococcus thermophilus* and deposited in the China General Microbiological Culture Collection Center on May 11, 2018, with a deposit number CGMCC No. 15752, where the China General Microbiological Culture Collection Center is located at No. 3, NO. 1 West Beichen Road, Chaoyang District, Beijing.

A classification of the LB42 strain involved in the present application is named *Lactobacillus delbrueckii* subsp. *Bulgaricus* and deposited in the China General Microbiological Culture Collection Center on May 11, 2018, with a deposit number CGMCC No. 15751, where the China General Microbiological Culture Collection Center is located at No. 3, NO. 1 West Beichen Road, Chaoyang District, Beijing.

BRIEF DESCRIPTION OF DRAWINGS

FIG. 1 is a statistical chart illustrating an abundance result of *Firmicutes* in ceca of each group of mice.

FIG. 2 is a statistical chart illustrating an abundance result of *Bacteroidetes* in ceca of each group of mice.

FIG. 3 is a statistical chart illustrating a ratio result of *Firmicutes* to *Bacteroidetes (F/B)* in ceca of each group of mice.

FIG. 4 is a statistical chart illustrating an abundance result of *Lachnospira* in ceca of each group of mice.

FIG. 5 is a statistical chart illustrating an abundance result of *Alistipes* in ceca of each group of mice.

FIG. 6 is a statistical chart illustrating an abundance result of *Acinetobacter* in ceca of each group of mice.

DETAILED DESCRIPTION

Embodiments are provided hereinafter to illustrate technical solutions of the present application. It is to be understood by those skilled in the art that the examples are intended to facilitate understanding of the present application and are not to be construed as limiting the present application.

The medium formulation involved in the following examples is as follows:

MRS medium (g/L): 10 g/L peptone, 10 g/L beef extract, 20 g/L glucose, 2 g/L sodium acetate, 5 g/L yeast powder, 2 g/L diammonium hydrogen citrate, 2.6 g/L $K_2PO_4 \cdot 3H_2O$, 0.1 g/L $MgSO_4 \cdot 7H_2O$, 0.05 g/L $MnSO_4$, 1 mL/L polysorbate 80 (Tween 80) and 0.5 g/L cysteine hydrochloride.

**Preparation Example 1**

This preparation example provides ten bacteria powder products. A specific preparation method is described below.

A BLa36 strain, a BL21 strain, a BBr60 strain, a BAC30 strain, a BI45 strain, a commercially available ATCC55814 strain, a commercially available ATCC15700 strain, a commercially available ATCC15703 strain, an ST81 strain and an LB42 strain were inoculated in MRS liquid media, respectively, cultured at 37°C for 24 h and activated twice continuously to obtain activation solutions, the activation solutions were inoculated in MRS liquid media at an inoculum volume of 3% (v/v) and cultured at 37°C for 18 h to obtain bacterial solutions, the bacterial solutions were centrifuged at 4000 rpm at 4°C for 5 min and filtered to obtain strains, and the strains were mixed with a protectant (20% skimmed milk) with a mass 5 times of the strain and freeze dried to obtain a BLa36 bacteria powder, a BL21 bacteria powder, a BBr60 bacteria powder, a BAC30 bacteria powder, a BI45 bacteria powder, an ATCC55814 bacteria powder, an ATCC15700 bacteria powder, an ATCC15703 bacteria powder, an ST81 bacteria powder and an LB42 bacteria powder. After a test, the numbers of viable bacteria were $5 \times 10^{11}$ CFU/g, $1 \times 10^{12}$ CFU/g, $1 \times 10^{11}$ CFU/g, $1 \times 10^{12}$ CFU/g, $6 \times 10^{11}$ CFU/g, $1 \times 10^{11}$ CFU/g, $5 \times 10^{11}$ CFU/g, $1 \times 10^{12}$ CFU/g, $1 \times 10^{11}$ CFU/g and $1 \times 10^{11}$ CFU/g for later use.

**Preparation Example 2**

This preparation example provides the composite *Bifidobacterium* inoculant products below.

(1) Five-bacteria compound probiotic agent 1: the BLa36 bacteria powder, the BL21 bacteria powder, the BBr60 bacteria powder, the BAC30 bacteria powder and the BI45 bacteria powder were uniformly mixed at a mass ratio of 2:2:2:1:1 to obtain the five-bacteria compound probiotic agent 1.

(2) Five-bacteria compound probiotic agent 2: the BLa36 bacteria powder, the BL21 bacteria powder, the BBr60 bacteria powder, the BAC30 bacteria powder and the BI45 bacteria powder were uniformly mixed at a mass ratio of 1:1:2:1:1 to obtain the five-bacteria compound probiotic agent 2.

(3) Five-bacteria compound probiotic agent 3: the BLa36 bacteria powder, the commercially available ATCC55814 bacteria powder, the commercially available ATCC15700 bacteria powder, the commercially available ATCC15703 bacteria powder and the BI45 bacteria powder were uniformly mixed at a mass ratio of 2:2:2:1:1 to obtain the five-bacteria compound probiotic agent 3.

(4) Four-bacteria compound probiotic agent 1: the BL21 bacteria powder, the BBr60 bacteria powder, the BAC30 bacteria powder and the BI45 bacteria powder were uniformly mixed at a mass ratio of 2:2:1:1 to obtain the four-bacteria compound probiotic agent 1.

(5) Four-bacteria compound probiotic agent 2: the BLa36 bacteria powder, the BBr60 bacteria powder, the BAC30 bacteria powder and the BI45 bacteria powder were uniformly mixed at a mass ratio of 2:2:1:1 to obtain the four-bacteria compound probiotic agent 2.

(6) Four-bacteria compound probiotic agent 3: the BLa36 bacteria powder, the BL21 bacteria powder, the BAC30 bacteria powder and the BI45 bacteria powder were uniformly mixed at a mass ratio of 2:2:1:1 to obtain the four-bacteria compound probiotic agent 3.

(7) Four-bacteria compound probiotic agent 4: the BLa36 bacteria powder, the BL21 bacteria powder, the BBr60 bacteria powder and the BI45 bacteria powder were uniformly mixed at a mass ratio of 2:2:2:1 to obtain the four-bacteria compound probiotic agent 4.

(8) Four-bacteria compound probiotic agent 5: the BLa36 bacteria powder, the BL21 bacteria powder, the BBr60 bacteria powder and the BAC30 bacteria powder were uniformly mixed at a mass ratio of 2:2:2:1 to obtain the four-bacteria compound probiotic agent 5.

(9) Three-bacteria compound probiotic agent: the BLa36 bacteria powder, the BL21 bacteria powder and the BBr60 bacteria powder were uniformly mixed at a mass ratio of 1:1:1 to obtain the three-bacteria compound probiotic agent.

**Preparation Example 3**

This preparation example provides the basic fermentation inoculant products below.

(1) Basic fermentation inoculant 1: the ST81 bacteria powder and the LB42 bacteria powder were uniformly mixed at a mass ratio of 10:1 to obtain the basic fermentation inoculant 1.

(2) Basic fermentation inoculant 2: the ST81 bacteria powder and the LB42 bacteria powder were uniformly mixed at a mass ratio of 3:1 to obtain the basic fermentation inoculant 2.

**Example 1**

This example provides a yogurt product. A preparation method of the yogurt product is described below.

(1) White granulated sugar and composite prebiotics (xylooligosaccharides, fructooligosaccharides and isomaltooligosaccharides with equal mass proportions) were added to raw cow milk and fully dissolved, where the raw cow milk accounted for 94% of the total weight, the white granulated sugar accounted for 5%, and the composite prebiotics accounted for 1%. Then, the mixture was sterilized at 95°C for 15 min, quickly cooled to 40°C in cold water and used as a fermentation base for later use.

(2) The basic fermentation inoculant 1 prepared in Preparation Example 3 (inoculum volume: 30 g/T) and the five-bacteria compound probiotic agent 1 (inoculum volume: 100 g/T) prepared in Preparation Example 2 were inoculated in the above sterilized fermentation base and fermented at a fermentation temperature of 43°C. When the acidity reached 75°T or pH = 4.4, the fermentation was stopped, and the mixture was refrigerated at 4°C.

The viable bacteria count of *Bifidobacteria* in this yogurt was counted with reference to the method in QB/T 4575-2013. The result indicated that the total viable bacteria count of *Bifidobacteria* in this yogurt was $8 \times 10^8$ CFU/g.

**Example 2**

This example provides a yogurt product. A preparation method of the yogurt product is described below.

(1) White granulated sugar and composite prebiotics (xylooligosaccharides, fructooligosaccharides and isomaltooligosaccharides with equal mass proportions) were added to raw cow milk and fully dissolved, where the raw cow milk accounted for 94% of the total weight, the white granulated sugar accounted for 5%, and the composite prebiotics accounted for 1%. Then, the mixture was sterilized at 95°C for 15 min, quickly cooled to 40°C in cold water and used as a fermentation base for later use.

(2) The basic fermentation inoculant 2 prepared in Preparation Example 3 (inoculum volume: 30 g/T) and the five-bacteria compound probiotic agent 2 (inoculum volume: 100 g/T) prepared in Preparation Example 2 were inoculated in the above sterilized fermentation base and fermented at a fermentation temperature of 40°C. When the acidity reached 75°T or pH = 4.4, the fermentation was stopped, and the mixture was refrigerated at 4°C.

The viable bacteria count of *Bifidobacteria* in this yogurt was counted with reference to the method in QB/T 4575-2013. The result indicated that the total viable bacteria count of *Bifidobacteria* in this yogurt was $4 \times 10^8$ CFU/g.

**Comparative Example 1**

This comparative example provides a yogurt product. The preparation method of the yogurt product differs from that of Example 1 only in that the five-bacteria compound probiotic agent 1 prepared in Preparation Example 2 was replaced with an equal amount of the four-bacteria compound probiotic agent 1 prepared in Preparation Example 2. Other conditions remained unchanged.

**Comparative Example 2**

This comparative example provides a yogurt product. The preparation method of the yogurt product differs from that of Example 1 only in that the five-bacteria compound probiotic agent 1 prepared in Preparation Example 2 was replaced with an equal amount of the four-bacteria compound probiotic agent 2 prepared in Preparation Example 2. Other conditions remained unchanged.

**Comparative Example 3**

This comparative example provides a yogurt product. The preparation method of the yogurt product differs from that of Example 1 only in that the five-bacteria compound probiotic agent 1 prepared in Preparation Example 2 was replaced with an equal amount of the four-bacteria compound probiotic agent 3 prepared in Preparation Example 2. Other conditions remained unchanged.

**Comparative Example 4**

This comparative example provides a yogurt product. The preparation method of the yogurt product differs from that of Example 1 only in that the five-bacteria compound probiotic agent 1 prepared in Preparation Example 2 was replaced with an equal amount of the four-bacteria compound probiotic agent 4 prepared in Preparation Example 2. Other conditions remained unchanged.

**Comparative Example 5**

This comparative example provides a yogurt product. The preparation method of the yogurt product differs from that of Example 1 only in that the five-bacteria compound probiotic agent 1 prepared in Preparation Example 2 was replaced with an equal amount of the four-bacteria compound probiotic agent 5 prepared in Preparation Example 2. Other conditions remained unchanged.

**Comparative Example 6**

This comparative example provides a yogurt product. The preparation method of the yogurt product differs from that of Example 1 only in that the five-bacteria compound probiotic agent 1 prepared in Preparation Example 2 was replaced with an equal amount of the five-bacteria compound probiotic agent 3 prepared in Preparation Example 2. Other conditions remained unchanged.

**Comparative Example 7**

This comparative example provides a yogurt product. The preparation method of the yogurt product differs from that of Example 1 only in that the five-bacteria compound probiotic agent 1 prepared in Preparation Example 2 was replaced with an equal amount of the three-bacteria compound probiotic agent prepared in Preparation Example 2. Other conditions remained unchanged.

**Comparative Example 8**

This comparative example provides a yogurt product. The preparation method of the yogurt product differs from that of Example 1 only in that the five-bacteria compound probiotic agent 1 prepared in Preparation Example 2 was not inoculated in step (2). Other conditions remained unchanged.

**Test Example 1**

Evaluation of tolerance to *in vitro* gastric juice

(1) Preparation of solutions

Preparation of an electrolyte solution A: 0.064 g of potassium chloride, 0.015 g of potassium dihydrogen phosphate, 0.263 g of sodium bicarbonate, 0.345 g of sodium chloride, 0.003 g of magnesium chloride hexahydrate, 0.006 g of ammonium carbonate, 1.5 g of tryptone and 0.05 g of L-cysteine hydrochloride were weighed, 95 mL of distilled water was added for thorough dissolution, the pH was adjusted to 3.0, and the volume of the solution was precisely adjusted to 100 mL.

Preparation of an electrolyte solution B: 0.022 g of calcium chloride dihydrate was weighed, water was added for dissolution, and the volume of the solution was precisely adjusted to 100 mL.

Dissolving solution: 8.5 g of sodium chloride and 0.5 g of L-cysteine hydrochloride monohydrate were weighed, and 1000 mL of distilled water was added for thorough dissolution.

All of the above three solutions were sterilized under a high pressure at 121°C for 15 min for later use.

8.0 mL of the electrolyte solution A and 1.0 mL of the electrolyte solution B were taken and placed in a beaker, pepsin equivalent to 4000 U was added, a pH value was adjusted by using a 1 mol/L hydrochloric acid solution or a 1 mol/L sodium hydroxide solution according to different simulated gastric juice conditions in Table 1, the volume of the solution was precisely adjusted to 10 mL, and the solution was uniformly mixed and filtered by using a 0.22 μm sterile filtration membrane to prepare simulated gastric juice, which was prepared and used immediately.

Table 1

| Simulated Gastric Juice Condition | pH Value | Treatment Time of Simulated Gastric Juice (h) |
|---|---|---|
| standard simulated gastric juice | 3 | 2 |
| fasting simulated gastric juice | 2 | 0.5 |
| simulated gastric juice on a full stomach | 4 | 3 |

(2) Detection of *Bifidobacterium*

1.0 g of each of the yogurts prepared in Examples 1 and 2 and Comparative Examples 1 to 7 was weighed through an aseptic operation, placed in a sterile homogenization bag with 99 mL of the dissolving solution, mixed uniformly and fully and beaten by using a homogenizer at 10 times/second for 2 min until the sample was dispersed uniformly to prepare a sample bacterial solution, which was prepared and used immediately. A total concentration of *Bifidobacteria* in the sample bacterial solution was measured with reference to GB 4789.35-2023 and recorded as N1 (unit: CFU/mL).

5.0 mL of the simulated gastric juice and 5.0 mL of the sample bacterial solution were taken and placed in a 50 mL centrifuge tube and vortexed to be uniformly mixed. About 5 mL of mineral oil was slowly added above the solution by using a dropper to form an oil seal layer above the solution. The centrifuge tube was placed in a water bath cauldron at a constant temperature of 37°C and cultured for a specified treatment time. The mineral oil was removed by using the dropper. A total concentration of *Bifidobacteria* was measured and recorded as N1' (unit: CFU/mL).

The above operations were repeated twice. The measured results of the total concentration of *Bifidobacteria* before the treatment were recorded as N2 and N3, and the measured results of the total concentration of *Bifidobacteria* after the treatment were recorded as N2' and N3'.

The tolerance to the gastric juice was evaluated according to a survival rate of the strains. The calculation formula is as follows:

$$\text{survival rate} (\%) = (2N1'/N1 + 2N2'/N2 + 2N3'/N3)/3 \times 100\%$$

The results of the average survival rates of the groups are shown in Table 2:

Table 2

| Group | pH = 2 | pH = 3 | pH = 4 |
|---|---|---|---|
| Example 1 | 83.7% | 88.3% | 96.3% |
| Example 2 | 83.5% | 89.2% | 96.1% |
| Comparative Example 1 | 70.2% | 74.3% | 75.5% |
| Comparative Example 2 | 63.4% | 64.3% | 66.4% |
| Comparative Example 3 | 54.9% | 63.9% | 72.5% |
| Comparative Example 4 | 63.5% | 68.2% | 70.1% |
| Comparative Example 5 | 60.8% | 68.3% | 70.4% |

(continued)

| Group | pH = 2 | pH = 3 | pH = 4 |
|---|---|---|---|
| Comparative Example 6 | 55.6% | 57.9% | 63.1% |
| Comparative Example 7 | 49.2% | 51.6% | 53.2% |

As can be seen from the data results in Table 2, the yogurt product obtained through the fermentation of the combination of the composite *Bifidobacterium* inoculant and the basic fermentation inoculant involved in the present application has excellent tolerance to gastric acid, and the survival rate of *Bifidobacterium* can still reach 83.7% at pH = 2. Compared with the data in Comparative Examples 1 to 7, if the compounding manner of the strains in the composite *Bifidobacterium* inoculant is changed and the survival rate of *Bifidobacterium* in the acidic environment is significantly reduced, it indicates that the compounding manner of the strains in the composite *Bifidobacterium* inoculant affects the tolerance of the yogurt to the gastric acid.

(3) Detection of *Lactobacillus*

1.0 g of each of the yogurts prepared in Examples 1 and 2 and Comparative Examples 1 to 8 was weighed through an aseptic operation, placed in a sterile homogenization bag with 99 mL of the dissolving solution, mixed uniformly and fully and beaten by using a homogenizer at 10 times/second for 2 min until the sample was dispersed uniformly to prepare a sample bacterial solution, which was prepared and used immediately. A total concentration of *Lactobacilli* in the sample bacterial solution was measured with reference to GB 4789.35-2023 and recorded as N1 (unit: CFU/mL).
5.0 mL of the simulated gastric juice and 5.0 mL of the sample bacterial solution were taken and placed in a 50 mL centrifuge tube and vortexed to be uniformly mixed. About 5 mL of mineral oil was slowly added above the solution by using a dropper to form an oil seal layer above the solution. The centrifuge tube was placed in a water bath cauldron at a constant temperature of 37°C and cultured for a specified treatment time. The mineral oil was removed by using the dropper. A total concentration of *Lactobacilli* was measured and recorded as N1' (unit: CFU/mL).
The above operations were repeated twice. The measured results of the total concentration of *Lactobacilli* before the treatment were recorded as N2 and N3, and the measured results of the total concentration of *Lactobacilli* after the treatment were recorded as N2' and N3'.
The tolerance to the gastric juice was evaluated according to a survival rate of the strains. The calculation formula is as follows:

$$\text{survival rate } (\%) = (2N1'/N1 + 2N2'/N2 + 2N3'/N3)/3 \times 100\%$$

The results of the average survival rates of *Lactobacilli* (the strains in the composite *Bifidobacterium* inoculant and the basic fermentation inoculant are all *Lactobacilli*) in the groups are shown in Table 3:

Table 3

| Group | pH = 2 | pH = 3 | pH = 4 |
|---|---|---|---|
| Example 1 | 81.6% | 85.0% | 95.7% |
| Example 2 | 82.1% | 84.8% | 95.3% |
| Comparative Example 1 | 69.3% | 70.4% | 71.2% |
| Comparative Example 2 | 62.1% | 65.1% | 67.5% |
| Comparative Example 3 | 51.9% | 62.9% | 65.7% |
| Comparative Example 4 | 60.3% | 65.1% | 71.3% |
| Comparative Example 5 | 60.9% | 62.4% | 65.6% |
| Comparative Example 6 | 40.4% | 43.5% | 46.9% |
| Comparative Example 7 | 8.7% | 2.8% | 1.0% |

As can be seen from the data results in Table 3, the yogurt product obtained through the fermentation of the combination of the composite *Bifidobacterium* inoculant and the basic fermentation inoculant involved in the present application has excellent tolerance to gastric acid, and the survival rate of all *Lactobacilli* can still reach 81.6% at pH = 2, while the survival rate of *Lactobacillus* in the yogurt product obtained through the fermentation of only the basic fermentation inoculant is only 8.7% at pH = 2. Compared with the data in Comparative Examples 1 to 7, if the compounding manner of the strains in the composite *Bifidobacterium* inoculant is changed and the survival rate of *Lactobacillus* in the acidic environment is significantly reduced, it indicates that the compounding manner of the strains in the composite *Bifidobacterium* inoculant affects the tolerance of the yogurt to the gastric acid.

Test Example 2

Evaluation of effect on intestinal motility of mice

(1) Laboratory animals

120 8-week-old BALB/c male mice (20±2 g) were raised in a clean and quiet environment at a temperature of 20-24°C and a humidity of 50% to 60%. The mice were purchased from SPF (Beijing) Biotechnology Co., Ltd.

(2) Animal grouping and intervention manners

After a 7-day adaptation period for all of the mice, the mice were randomly divided into 12 groups: a blank control group NC, a model group MC and yogurt groups prepared in Examples 1 and 2 and Comparative Examples 1 to 8. On the first day of the experiment, the blank control group and the model group each were given 200 μL of normal saline through gavage, and other yogurt groups each were given 200 μL of yogurt through gavage. During the experiment, the groups were subjected to gavage once a day. The modeling manner is as follows: on the 15th to 17th days of the experiment, except for the blank control group, other groups of mice each were given 10 mg/kg.bw loperamide through gavage and given normal saline or yogurt through gavage after 4 h. The specific cases are shown in Table 4.

Table 4

| Grouping | Modeling Manner and Intervention Manner |
| --- | --- |
| Blank control group | normal saline |
| Model group | normal saline+loperamide |
| Example 1 group | yogurt in Example 1+loperamide |
| Example 2 group | yogurt in Example 2+loperamide |
| Comparative Example 1 group | yogurt in Comparative Example 1+loperamide |
| Comparative Example 2 group | yogurt in Comparative Example 2+loperamide |
| Comparative Example 3 group | yogurt in Comparative Example 3+loperamide |
| Comparative Example 4 group | yogurt in Comparative Example 4+loperamide |
| Comparative Example 5 group | yogurt in Comparative Example 5+loperamide |
| Comparative Example 6 group | yogurt in Comparative Example 6+loperamide |
| Comparative Example 7 group | yogurt in Comparative Example 7+loperamide |
| Comparative Example 8 group | yogurt in Comparative Example 8+loperamide |

(3) Evaluation of safety of mice

During the animal experiment, body weights and food intakes were measured twice a week to observe healthy states of the mice. The results show that before the mice are modeled, the body weight of each group of mice exhibits an upward trend, indicating that the intervention samples have no side effects on the weight gain of the mice, there is no significant difference in change of body weight of each group of mice and each group of mice gains a weight of 5% or more, indicating that the mice are healthy and grow well. A food utilization rate of each group of mice is similar to that of the control group, and the food utilization rate is higher than 3%, indicating that the food utilization of each group of mice is good.

(4) Evaluation of effects

(4.1) First melena discharge time

On the 18th day of the experiment, after fasting without water deprivation for 16 h, the control group was given normal saline through gavage, and other groups of mice were given loperamide through gavage. After 1 h, ink was given through gavage at 0.2 mL/mouse. Starting from giving ink through gavage, the first melena discharge time (min) of each mouse was recorded, and the first melena time of the last mouse in the model group was used as the termination time.
The statistical results are shown in Table 5. Compared with the blank control group, the first melena discharge time of mice in the model group is significantly prolonged, the first melena discharge time of each group of mice is shortened to varying degrees after the intervention of the yogurt in each group, and the effects of the Example 1 group and the Example 2 group have apparent advantages, indicating that the probiotic agent involved in the present application has effects of promoting intestinal peristalsis and loosening bowel to relieve constipation.

(4.2) Small intestine propulsion rate

On the 19th day of the experiment, the control group was given normal saline through gavage, and other groups of mice were given loperamide through gavage. After 1 h, all of the mice were given ink through gavage (0.2 mL/mouse). After 25 min, the mice were sacrificed. An intestinal tube with a superior end from the pylorus and an inferior end to the ileocecum was cut off, and the small intestine was gently pulled into a straight line. A length of the intestinal tube was measured as "a total length of the small intestine", and a length from the pylorus to the front edge of the ink was measured as "an ink propulsion length". Calculation was performed as follows: small intestine propulsion rate (%) = ink propulsion length/total length of small intestine$\times$ 100%.
The statistical results are shown in Table 5. Compared with the blank control group, the small intestine propulsion rate of the model group is significantly reduced, the small intestine propulsion rate of each group of mice is restored to varying degrees after the intervention of the yogurt in each group, and the restoration effects of the Example 1 group and the Example 2 group have apparent advantages, indicating that the probiotic agent involved in the present application has effects of promoting intestinal peristalsis and loosening bowel to relieve constipation.

Table 5

| Group | First Melena Discharge Time (min) | Small Intestine Propulsion Rate (%) |
|---|---|---|
| Blank control group | 60.3 | 93.5 |
| Model group | 173.6 | 35.0 |
| Example 1 group | 104.0 | 76.2 |
| Example 2 group | 109.2 | 74.9 |
| Comparative Example 1 group | 132.8 | 50.3 |
| Comparative Example 2 group | 119.5 | 49.7 |
| Comparative Example 3 group | 142.1 | 49.3 |
| Comparative Example 4 group | 138.8 | 48.0 |
| Comparative Example 5 group | 142.1 | 53.4 |
| Comparative Example 6 group | 119.9 | 49.5 |
| Comparative Example 7 group | 127.7 | 44.0 |
| Comparative Example 8 group | 150.8 | 38.3 |

(4.3) Analysis of gastrointestinal regulatory peptides

After the experiment was finished, the mice were dissected. Blood was collected, serums were separated, and contents of four gastrointestinal regulatory peptides (Gas, SP, VIP and ET-1) in the serums of the mice were measured by using an ELISA kit. The statistical results are shown in Table 6. Gas and SP are excitatory peptide neurotransmitters, while ET-1 and VIP are inhibitory peptide neurotransmitters. After the intervention of each group, compared with the model group, both Gas and SP are improved, while ET-1 and VIP are significantly reduced, and the effects of the Example 1 group and the Example 2 group are the best, indicating that the yogurt prepared by using the probiotic agent involved in the present application can promote intestinal peristalsis.

Table 6

| Group | Gas (pg/mL) | SP (pg/mL) | VIP (pg/mL) | ET-1 (pg/mL) |
|---|---|---|---|---|
| Blank control group | 150.72 | 1003.92 | 130.19 | 139.83 |
| Model group | 100.39 | 520.29 | 258.78 | 170.02 |
| Example 1 group | 139.03 | 863.21 | 160.34 | 142.19 |
| Example 2 group | 136.99 | 903.18 | 153.98 | 143.84 |
| Comparative Example 1 group | 129.64 | 793.28 | 172.29 | 153.33 |
| Comparative Example 2 group | 128.30 | 830.16 | 174.49 | 152.69 |
| Comparative Example 3 group | 130.02 | 749.28 | 169.11 | 162.22 |
| Comparative Example 4 group | 124.88 | 693.35 | 183.29 | 160.01 |
| Comparative Example 5 group | 116.39 | 642.10 | 184.65 | 150.09 |
| Comparative Example 6 group | 113.25 | 577.31 | 204.30 | 152.93 |
| Comparative Example 7 group | 112.49 | 620.10 | 230.71 | 162.09 |
| Comparative Example 8 group | 102.34 | 559.33 | 259.83 | 168.69 |

(4.4) Analysis of inflammatory factors

Contents of inflammatory factors (IL-6, IL-10 and TNF-$\alpha$) in serums of the mice were measured by using an ELISA kit. The statistical results are shown in Table 7. After the intervention of each group, compared with the model group, the content of the anti-inflammatory factor IL-10 is improved, while the contents of the proinflammatory factors IL-6 and TNF-$\alpha$ are reduced, and the effects of the Example 1 group and the Example 2 group are the best.

Table 7

| Group | IL-10 (pg/mL) | IL-6 (pg/mL) | THF-$\alpha$ (pg/mL) |
|---|---|---|---|
| Blank control group | 370.98 | 130.04 | 101.38 |
| Model group | 210.82 | 160.96 | 255.83 |
| Example 1 group | 363.29 | 134.01 | 118.86 |
| Example 2 group | 358.01 | 139.95 | 127.53 |
| Comparative Example 1 group | 299.01 | 144.09 | 170.55 |
| Comparative Example 2 group | 311.83 | 153.91 | 139.65 |
| Comparative Example 3 group | 329.30 | 148.87 | 169.48 |
| Comparative Example 4 group | 331.28 | 147.50 | 143.92 |
| Comparative Example 5 group | 270.03 | 159.20 | 163.27 |
| Comparative Example 6 group | 255.65 | 154.39 | 189.05 |
| Comparative Example 7 group | 230.20 | 156.82 | 204.38 |
| Comparative Example 8 group | 200.05 | 162.01 | 236.60 |

(4.5) Analysis of metagenomes of intestinal floras

Ceca of mice in the blank control group, the model group, the Example 1 group and the Comparative Example 8 group were cut off, and metagenomes of intestinal floras were analyzed through 16S rDNA.

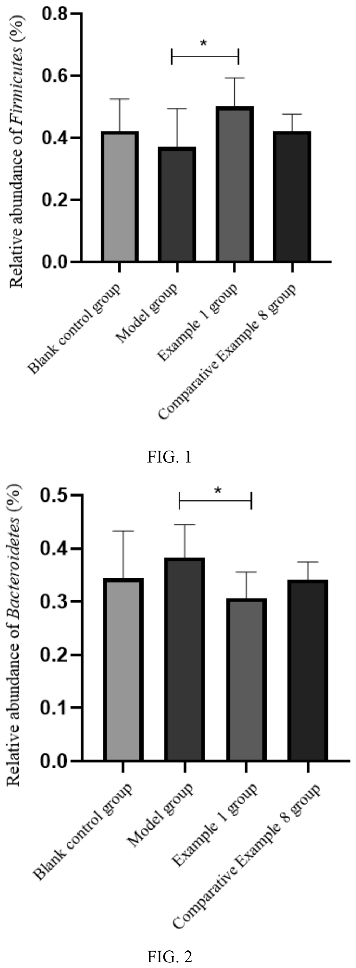

*Firmicutes* and *Bacteroidetes* account for the main proportion of the intestinal flora. These two floras and a ratio of *Firmicutes* to *Bacteroidetes* (F/B) were analyzed in detail. F/B is related to the maintenance of the *in vivo* balance. The statistical results are shown in FIGS. 1 to 3. Compared with the blank control group, the number of *Firmicutes* in the model group is decreased, and the number of *Bacteroidetes* in the model group is increased, indicating that with the constipation of the mice, beneficial floras in the ceca are decreased and the numbers of some pathogens are increased. Compared with the model group, the number of *Firmicutes* in the intervening group is increased, the number of *Bacteroidetes* in the intervening group is decreased significantly, and F/B is higher than that of the model group, indicating that the intake of the probiotic yogurt of the present application has a promoting effect on the balance of the intestinal floras.

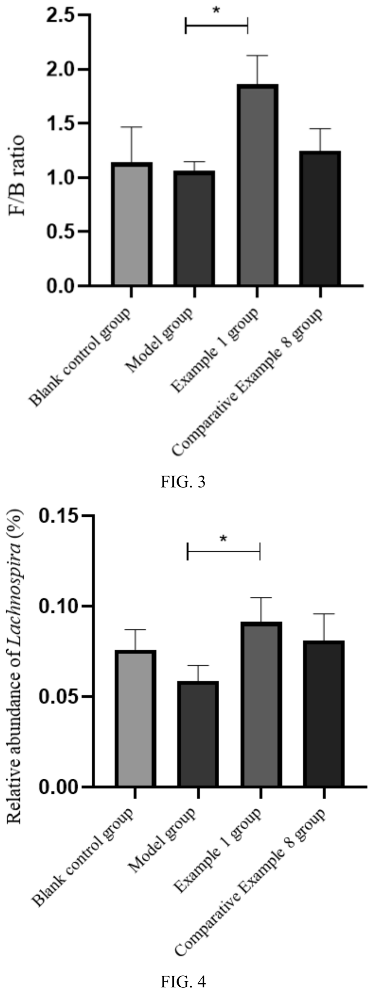

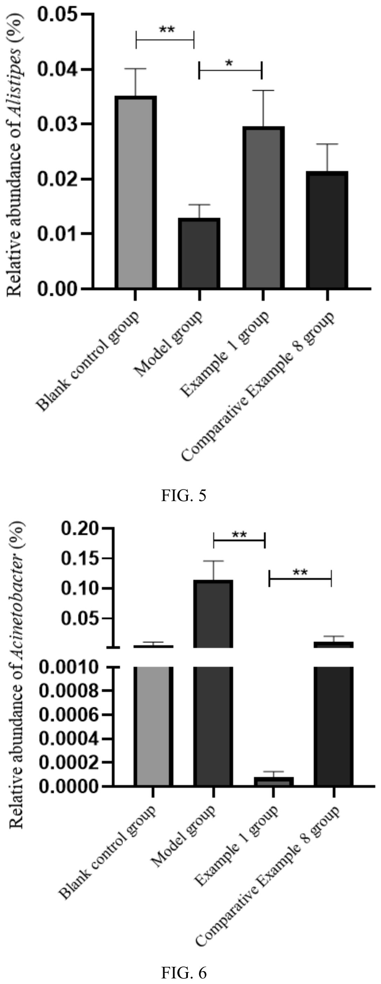

*Lachnospira* can hydrolyze starch and other saccharides to produce butyrate and other short-chain fatty acids. *Alistipes* helps human digestion, nutrient absorption and regulation. *Acinetobacter* belongs to gram-negative bacteria and causes infection when the resistance of the body is reduced. *Acinetobacter* may colonize in human skin, a wound, a respiratory tract and a gastrointestinal tract and may also exist in an oral biofilm. *Acinetobacter* is easy to cause pneumonia after being inhaled into the lower respiratory tract. Genus levels of the three floras were subjected to statistics. As shown in FIGS. 4 to 6, compared with the model group, the numbers of *Lachnospira* and *Alistipes* are increased and the numbers of *Acinetobacter* are decreased in the Example 1 group and the Comparative Example 8 group, and the effect of the Example 1 group is significantly more excellent, indicating that the intake of the probiotic yogurt of the present application can reduce the number of harmful bacteria in the intestinal tract and increase the number of beneficial bacteria in the intestinal tract, and at the genus level, also indicating that the balance of the intestinal floras can be improved.

The applicant has stated that although the technical solutions of the present application are described through the examples described above, the present application is not limited to the examples described above, which means that the implementation of the present application does not necessarily depend on the examples described above. It should be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

Although preferred embodiments of the present application are described above in detail, the present application is not limited to details of the preceding embodiments, and various simple modifications can be made to the technical solutions of the present application without departing from the technical concept of the present application. These simple modifications are all within the protection scope of the present application.

Additionally, it is to be noted that if not in collision, specific technical features described in the preceding embodiments can be combined in any suitable manner. To avoid unnecessary repetition, various possible combination manners are no longer described in the present application.

**Claims**

1. A composite *Bifidobacterium* inoculant, whose strain is composed of a *Bifidobacterium animalis* subsp. *lactis* BLa36 strain with a deposit number CGMCC No. 24029, a *Bifidobacterium longum* BL21 strain with a deposit number CGMCC No. 10452, a *Bifidobacterium breve* BBr60 strain with a deposit number CGMCC No. 12915, a *Bifidobacterium adolescentis* BAC30 strain with a deposit number CGMCC No. 19884 and a *Bifidobacterium infantis* BI45 strain with a deposit number CGMCC No. 15134.

2. The composite *Bifidobacterium* inoculant according to claim 1, wherein a ratio of a viable bacteria count of the BLa36 strain to a viable bacteria count of the BL21 strain to a viable bacteria count of the BBr60 strain to a viable bacteria count of the BAC30 strain to a viable bacteria count of the BI45 strain is (1-10):(1-10):(1-10):(1-5):(1-5).

3. The composite *Bifidobacterium* inoculant according to claim 1, wherein a total viable bacteria count in the composite *Bifidobacterium* inoculant is not less than $1 \times 10^{11}$ CFU/mL or $1 \times 10^{11}$ CFU/g.

4. The composite *Bifidobacterium* inoculant according to claim 1, wherein a dosage form of the composite *Bifidobacterium* inoculant comprises a solution, a freeze-dried powder, a capsule, a tablet or a granule.

5. A composite probiotic agent, comprising the composite *Bifidobacterium* inoculant according to any one of claims 1 to 4 and a basic inoculant, wherein a strain in the basic inoculant is composed of a *Streptococcus thermophilus* ST81 strain with a deposit number CGMCC No. 15752 and a *Lactobacillus delbrueckii* subsp. *Bulgaricus* LB42 strain with a deposit number CGMCC No. 15751.

6. The composite probiotic agent according to claim 5, wherein a ratio of a viable bacteria count of the ST81 strain to a viable bacteria count of the LB42 strain is 30:(0.001-10);

   a total viable bacteria count in the basic inoculant is not less than $1 \times 10^{11}$ CFU/mL or $1 \times 10^{11}$ CFU/g;
   a mass ratio of the composite *Bifidobacterium* inoculant to the basic inoculant is (10-300):(10-40); and
   a dosage form of the composite probiotic agent comprises a solution, a freeze-dried powder, a capsule, a tablet or a granule.

7. Use of the composite *Bifidobacterium* inoculant according to any one of claims 1 to 4 or the composite probiotic agent according to any one of claims 5 and 6 in yogurt fermentation.

8. The use according to claim 7, wherein a method of the yogurt fermentation comprises:

   (1) mixing raw cow milk with composite prebiotics, sterilizing and cooling, and using the mixture as a fermentation base; and
   (2) inoculating the composite probiotic agent in the fermentation base for fermentation.

9. The use according to claim 8, wherein the composite prebiotics comprise any one or a combination of at least two of fructooligosaccharides, galactooligosaccharides, xylooligosaccharides, isomaltooligosaccharides, soybean oligo-saccharides, inulins, polydextrose, $\alpha$-whey protein or lactoferrin;

   preferably, the composite prebiotics are 0.01% to 1% of the fermentation base;
   preferably, an inoculation amount of the composite probiotic agent is 20-300 g/T;
   preferably, the fermentation is performed at a temperature of 40-43°C for 5-10 h, and when an acidity reaches 70-80°T or pH = 4.3-4.5, the fermentation reaches an end point.

10. Use of the composite *Bifidobacterium* inoculant according to any one of claims 1 to 4 or the composite probiotic agent according to any one of claims 5 and 6 in preparing a product for promoting intestinal peristalsis and promoting intestinal health.

FIG. 1

FIG. 2

FIG. 3

FIG. 4

FIG. 5

FIG. 6

## INTERNATIONAL SEARCH REPORT

| International application No. |
|---|
| **PCT/CN2025/094593** |

| A. | CLASSIFICATION OF SUBJECT MATTER |
|---|---|

C12N1/20(2006.01)i; A23C9/123(2006.01)i; A61K35/745(2015.01)i; A61P1/00(2006.01)i

According to International Patent Classification (IPC) or to both national classification and IPC

| B. | FIELDS SEARCHED |
|---|---|

Minimum documentation searched (classification system followed by classification symbols)

IPC: C12N, A23C, A61K, A61P

Documentation searched other than minimum documentation to the extent that such documents are included in the fields searched

Electronic data base consulted during the international search (name of data base and, where practicable, search terms used)

VCN, VEN, WPABSC, ENTXT, OETXT, CNKI, PubMed, Elsevier Science, ISI WEB of Science: 菌剂, 酸奶发酵, 动物双歧杆菌乳亚种, 长双歧杆菌, 短双歧杆菌, 青春双歧杆菌, 婴儿双歧杆菌, microbial agent, yogurt fermentation, Bifidobacterium animalis subsp lactis, Bifidobacterium longum, Bifidobacterium breve, Bifidobacterium adolescentis, Bifidobacterium infantis, CGMCC No.24029, CGMCC No.10452, CGMCC No.12915, CGMCC No.19884, CGMCC No.15134.

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 118652824 A (WECARE PROBIOTIC (SUZHOU) CO., LTD.) 17 September 2024 (2024-09-17)<br>see abstract and claims 1-10 | 1-10 |
| A | CN 118272282 A (WECARE PROBIOTIC (SUZHOU) CO., LTD.) 02 July 2024 (2024-07-02)<br>see abstract, claims 1-10, and description, paragraphs 34-46 | 1-10 |
| A | CN 114350577 A (WECARE PROBIOTIC (SUZHOU) CO., LTD.) 15 April 2022 (2022-04-15)<br>see abstract, claims 1-10, and embodiments 1-11 | 1-10 |
| A | CN 118048280 A (WECARE PROBIOTIC (SUZHOU) CO., LTD.) 17 May 2024 (2024-05-17)<br>see abstract, claims 1-10, and description, paragraphs 3 and 7 | 1-10 |

☑ Further documents are listed in the continuation of Box C. ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **28 July 2025** | **19 August 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2025/094593** |

### C.    DOCUMENTS CONSIDERED TO BE RELEVANT

| Category* | Citation of document, with indication, where appropriate, of the relevant passages | Relevant to claim No. |
|---|---|---|
| A | CN 115895979 A (SUZHOU WEIKE LIFE TECHNOLOGY CO., LTD.) 04 April 2023 (2023-04-04)<br>  see abstract and claims 1-10 | 1-10 |
| A | CN 116445356 A (WECARE PROBIOTIC (SUZHOU) CO., LTD.) 18 July 2023 (2023-07-18)<br>  see abstract and claims 1-10 | 1-10 |
| A | CN 118497046 A (WECARE PROBIOTIC (SUZHOU) CO., LTD.) 16 August 2024 (2024-08-16)<br>  see abstract and claims 1-10 | 1-10 |
| A | CN 117568244 A (WECARE PROBIOTIC (SUZHOU) CO., LTD.) 20 February 2024 (2024-02-20)<br>  see abstract and claims 1-10 | 1-10 |
| A | WO 2023185338 A1 (BEIJING SANYUAN FOODS CO., LTD.) 05 October 2023 (2023-10-05)<br>  see abstract, and claims 1-11 | 1-10 |
| A | SON, J. K. et al. "Fermentation and Quality Characteristics of Yogurt Treated with Bifidobacterium longum"<br>*Nutrients*, Vol. 15, No. 15, 07 August 2023 (2023-08-07),<br>  see abstract | 1-10 |

Form PCT/ISA/210 (second sheet) (July 2022)

INTERNATIONAL SEARCH REPORT
Information on patent family members

International application No.

**PCT/CN2025/094593**

| Patent document cited in search report | | | Publication date (day/month/year) | Patent family member(s) | Publication date (day/month/year) |
|---|---|---|---|---|---|
| CN | 118652824 | A | 17 September 2024 | None | |
| CN | 118272282 | A | 02 July 2024 | None | |
| CN | 114350577 | A | 15 April 2022 | None | |
| CN | 118048280 | A | 17 May 2024 | None | |
| CN | 115895979 | A | 04 April 2023 | None | |
| CN | 116445356 | A | 18 July 2023 | None | |
| CN | 118497046 | A | 16 August 2024 | None | |
| CN | 117568244 | A | 20 February 2024 | None | |
| WO | 2023185338 | A1 | 05 October 2023 | None | |

Form PCT/ISA/210 (patent family annex) (July 2022)

**EP 4 726 025 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 4789352023 A